Europäisches Patentamt

European Patent Office

Office européen des brevets

(11) Publication number: **0 452 490 A1**

# EUROPEAN PATENT APPLICATION
## published in accordance with Art. 158(3) EPC

(21) Application number: 89909257.1

(22) Date of filing: 25.04.89

(86) International application number:
PCT/SU89/00110

(87) International publication number:
WO 90/12616 (01.11.90 90/25)

(51) Int. Cl.⁵: **A61M 25/00, A61B 17/12**

(43) Date of publication of application:
**23.10.91 Bulletin 91/43**

(84) Designated Contracting States:
**DE FR GB IT SE**

(71) Applicant: **KIEVSKY NAUCHNO-ISSLEDOVATELSKY INSTITUT NEIROKHIRURGII**
**ul. Manuilskogo, 32**
**Kiev, 252655(SU)**

(72) Inventor: **SCHEGLOV, Viktor Ivanovich**
**ul. Belorusskaya, 15b-51**
**Kiev, 252119(SU)**
Inventor: **SAVENKO, Alexandr Grigorievich**

**deceased(SU)**

(74) Representative: **Lerwill, John et al**
**A.A. Thornton & Co. Northumberland House**
**303-306 High Holborn**
**London, WC1V 7LE(GB)**

(54) **OCCLUDING DEVICE.**

(57) An occluding device comprising a dismountable inflatable balloon (1) provided with a X-ray contrast mark (8) and connected, through a sphincter, to the working end of a catheter consisting of a conical bell mouth (5), whose enlarged part faces the hollow of the balloon (1).

FIG. 1

EP 0 452 490 A1

Technical Field

The invention relates generally to medicine, more specifically to radiological surgery, and is concerned particularly with occluding devices applicable for treatment of the various vasculopathies, such as arterial and other intracranial aneurysms and aneurysmal vasculostomies of diverse localisation and size, involving reconstructive vasoplastic surgery.

Prior Art

At present one of the most widespread diseases affecting human beings are vascular diseases of the brain, heart and other organs. It is surgical treatment that proves to be the most efficacious way of remedying the vascular diseaes. However, though being an efficient way of treatment of vasculopathies, surgical interference involves much traumatism, especially in surgery on cerebral vessels, severe postoperative complications, as well as its being a sophisticated and rather costly procedure.

Of late radiological (or endovascular) surgery has been gaining extensive application. Use of detachable balloons-catheters in endovascular surgery enables one to exclude the aneurysm from the blood flow (i.e., to occlude or obturate its sac) without cutting the cranium open and with the brain and its surrounding structures remaining intact, as well as with retention of patency of the intracranial arteries, including those carrying the aneurysm. In this case the surgery inflicts a minimized operative trauma upon the brain and a minimum degree of interference with the cerebral circulation and the brain functions. Besides, the operating time and the duration of the postoperative period are cut down considerably. However, endovascular exclusion of aneurysms with the aneurysm-carrying vessel remaining passable, special difficulties are encountered when inserting the occluding device into the aneurysmal sac and detaching the removable balloon from the catheter.

In a majority of cases intravascular treatment of aneurysms is completed by an occlusion of the parent vessel at the level of the aneurysmal opening with the aid of detachable balloons-catheters. As a rule, to detach the catheter from the balloons requires much effort to be applied, which might result in the rupture of the vessel or tearing of minor vessels off from major arteries and, hence, eventuate most frequently in severe postoperative complications.

Imperfection of the currently used radiological-surgery instruments hampers the solution of one of the most important problems in medicinal practice, i.e. treatment of vasculopathies and necessitates the development of basically novel occluding devices for performing the surgical procedures mentioned above.

Known in the present state of the art are quite a number of occluding devices for performing radiological-surgery procedures on vessels, e.g. a catheter with a detachable balloon (GB, A, 2,045,621).

The occluding device, according to the aforesaid British Patent, incorporates a detachable latex balloon communicating with a PVC catheter through a silicone tube. The balloon, the tube and the catheter are bonded together by means of an adhesive, and the spots of adhesive bonding are coated with epoxy resin. A loop-shaped electrode is placed on the adapter tube and is connected to the source of current through wire conductors.

The balloon is detached from the catheter by burning out the connecting tube, using the loop-electrode to which radio-frequency current is applied. The connecting wires run inside the catheter, and the loop-electrode is insulated from the surrounding medium by means of an additional dielectric sleeve.

The occluding device of the aforementioned construction is inserted, through a puncture needle, into the affected artery and brought to a pathologically changed arterial portion, followed by complete occlusion of the affected vessel. Then, the balloon is filled with a rapidly solidifying matter, and the catheter is detached from the balloon as soon as said matter polymerizes, by burning out the connecting tube. Such a construction arrangement of the occluding device provides for reliable affixing of the balloon on the catheter and its fairly facile detachment therefrom. However, rigid construction of the occlusion device and its inadequate manoeuvrability prevent it from being inserted into the aneurysmal sac and into the arteries of the 2nd and 3rd orders, while detachment of the catheter from the balloons by virtue of electric current is fraught with such complications as blood coagulation, microthrombosis and disturbed blood micro-circulation.

One of the most commonly known and most extensively used occluding devices is one (FR, A, 2,383,673), which is essentially a balloon-catheter, comprising a detachable distensible balloon provided with a radiopaque marker, which is in fact a metallic clip located in the thickened wall of the balloon opposite to the sphincter, whose edges are curved inwards the balloon fitted over the catheter working end and fixed in place by means of 5 or 6 turns of a latex microthread. After the affected vessel (aneurysm) has been excluded from the blood flow the balloon is filled with a rapidly solidifying matter, while the catheter is detached from the balloon by pulling it out. While so doing, the balloon is held in position with the aid of a second, auxiliary larger-diameter catheter fitted onto the main working catheter. The catheters are displaced in the opposite directions, i.e., the main catheter is taken out, while the auxiliary catheter is forwarded towards the balloon. Using such manipulations, the balloon is disengaged from the catheter, whereupon both catheters are withdrawn from the vessel operated upon. When the catheter is removed from the balloon the sphincter is turned inside out and may thereby close partly the lumen of the aneurysm-carrying vessel. To detach the balloon from the catheter, one should apply much effort, which is fraught with a danger of traction of minor vessels, their rupture or separation from the surrounding structures. In order to reduce the effort to be applied for detaching the balloon from the catheter, the sphicter is to be made thinned, which involves its addition fixing on the catheter. However, such additional fixation is difficult to perform and proves to be low reliable so that spontaneous detachment of the balloon from the catheter is possible, which might result in severe postoperative events caused by embolism of the vitally important vessels. There should also be considered as disadvantages of said construction its rigidity, unreliable fixation of the balloon on the catheter, too high an effort necessary for disengaging the balloon from the catheter, which narrows the firld of application of occluding devices of the aforesaid construction. The devices are largely applicable for stationary occlusion of aneurysm-carrying vessels, which might lead to a drastic change in the blood supply and to grave postoperative complications.

An occluding device closest to that disclosed in this in this invention is the one described in US Patent A, 4,282,875. The device comprises a detachable balloon with a thickened sphincter, and a catheter whose distal end has a spheroidal bulb. The sphincter orifice encompasses tightly the catheter end extending into the balloon, thus fixing the balloon on the catheter reliability.

However, the construction of the aforesaid occluding device involves much effort to be applied for detaching the balloon from the catheter, which might result in vessel rupture or in tearing of the minor subcortical arterial vessels from the vessel operated upon, which in turn might cause such postoperative complications as ischemia and necrosis of tissues and organs of different degrees, as well as speech and motor disturbances.

Practical application of the aforediscussed balloons-catheters is restricted due to their rigid construction, which prevents them from penetration through the flexures of the internal carotid artery and into the aneurysmal sac, while too great forces developed during detachment of the catheter from the balloon might involve dangerous postoperative complications. The balloon has such a structure that the balloon-to-catheter joint interferes with performance of efficacious radiological-surgery procedures for treatment of cerebral vasculopathies and those of various organs and tissues.

Disclosure of the Invention

It is a primary and essential object of the present invention to provide such an occluding device that would make it possible to minimize an effort to be applied for detaching the balloon from the catheter and be instrumental in teliably fixing the balloon on the catheter so as t to prevent its spontaneous detachment, all this being attained due to an appropriately engineered balloon-to--catheter joint,

The aforesaid object is accomplished due to the fact that in an occluding device, comprising a detachable distensible balloon provided with a radiopaque marker and communicating with the catheter working end through a sphincter, according to the invention, the catheter working end is shaped as a bellmouth with its flaring-out portion facing inwards the balloon.

Such a construction arrangement makes it possible to carry out an endovascular surgical procedure without any feat for spontaneous disengagement of the balloon from the catheter, at the same time enabling the catheter to be withdrawn the balloon without applying extra efforts.

It is practicable to provide the occluding device with a sealing ring that encompasses the catheter working end from the inner side of the balloon, while the greater base of the bellmouth may be bevelled at an angle to the catheter longitudinal axis.

Such a construction arrangement is instrumental in a reduced force required to disengage the catheter from the balloon, this being due to a decreased amount of compression exerted by the sphincter on the catheter and a displacement of the packing ring opening with respect to the sphincter, thus preventing the filler of the balloon from getting into the vascular tract. The construction of such an occluding device provides for its reliable fixing during treatment of aneurysms affecting the cavernous segment of the carotid artery, the vessels of the abdominal organs, etc.

It is expedient that the sealing ring be held in place to the sphincter inner surface and that the hole in the ring be offset with respect to the sphincter opening by an amount exceeding the sphincter diameter.

Such a construction arrangement makes it possible to reliably fix the balloon on the catheter and to ensure against spontaneous detachment of the balloon from the catheter, while minimizing an effort required for disengagement of the catheter from the balloon after the latter has been filled with a rapidly solidifying matter. This also renders the balloon more hermetically sealed and rules out a possibility of escaping of the rapidly solidifying matter from the balloon and its getting into the vascular tract after catheter disengagement.

It is practicable to provide the occluding device with a disk encompassing the catheter working end from the inner side of the balloon and filled with a liquid. Such a construction feature enables one to minimize the effort required for detaching the catheter from the balloon so as to reliably exclude aneurysms from the blood flow within an acute period involving repeated hemorrhage, as well as saccular aneurysms, traumatic aneurysms of the cerebral vessels, and to rule out completely tearing of blood vessels off the organs and tissues.

Summary of the Drawings

In what follows the present invention is illustrated by a description of some specific exemplary embodiments thereof with reference to the accompanying drawings.

Fig. 1 is a general longitudinal-section view of an occluding device, according to the invention.

Fig. 2 is a general longitudinal-section view of an occluding device featuring a sealing ring and a bevelled bellmouth of the catheter working end, according to the invention.

Fig. 3 is a general view of a sealing ring, according to the invention.

Fig. 4 is a longitudinal-section view of an occluding device showing a sealing ring held in place thereto, according to the invention.

Fig. 5 is a longitudinal-section view of an occluding device with a fixing disk, according to the invention; and

Fig. 6 is a view of an occluding device while in operation, according to the invention,

Preferred Embodiment of the Invention

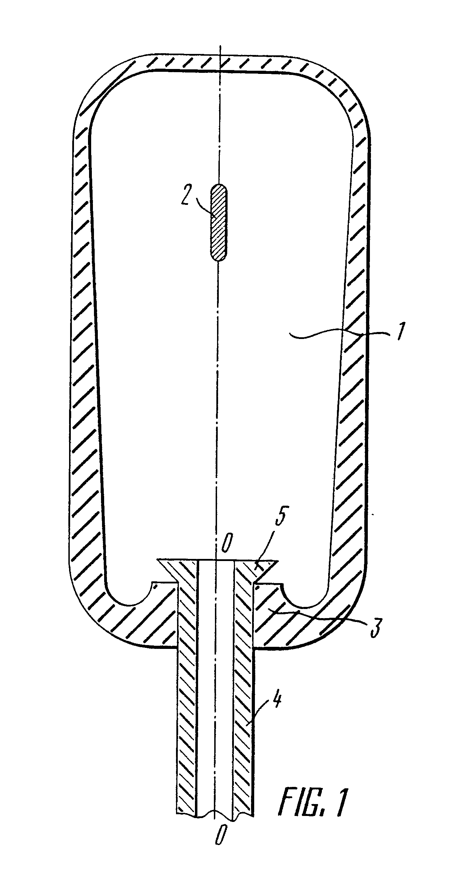

The occluding device, as disclosed in the present invention, comprises a detachable distensible balloon 1 (Fig. 1) provided with a radiopaque marker 2 and communicating, via a sphincter 3, with a catheter 4 whose working end is provided with a bellmouth 5 the flaring-out portion of which faces inwards the balloon 1.

The balloon 1 is inserted, via a puncture needle (omitted in the Drawing), into an arterial vessel and, while utilizing specific features of hemodynamics, is brought to the aneurysmal sac. The position assumed by the balloon 1 is monitored with-the aid of roentgenotelevision engineering means, against the radiopaque marker 4, using a radiopaque substance. Then, the balloon 1 is filled with a rapidly solidifying matter through the catheter 4, with the result that the balloon 1 is distended unevenly, though the degree of compression exerted by the sphincter on the catheter is decreased. An increasing gap between the inner face of an opening in the sphincter 3 and the outer wall of the catheter 4 enables the catheter portion located in the opening, to be wetted with blood when an attempt is made to withdraw the catheter 4, which reduces considerably an effort required to detach the catheter from the balloon and allows one to easily withdraw the catheter from the balloon. Then, the sphincter 3 is drawn up tight, thus closing the balloon 1 reliably. The working end of the catheter 4 is shaped as a bellmouth 5 facing inwards the balloon 1.

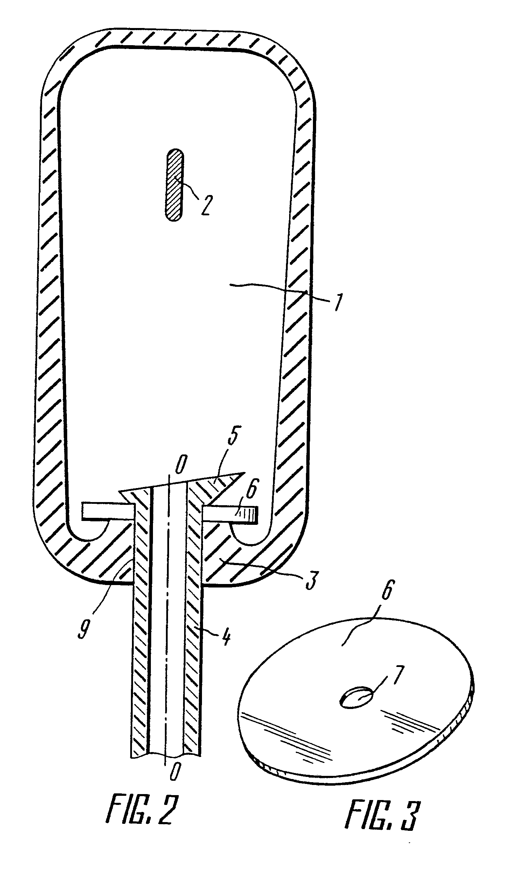

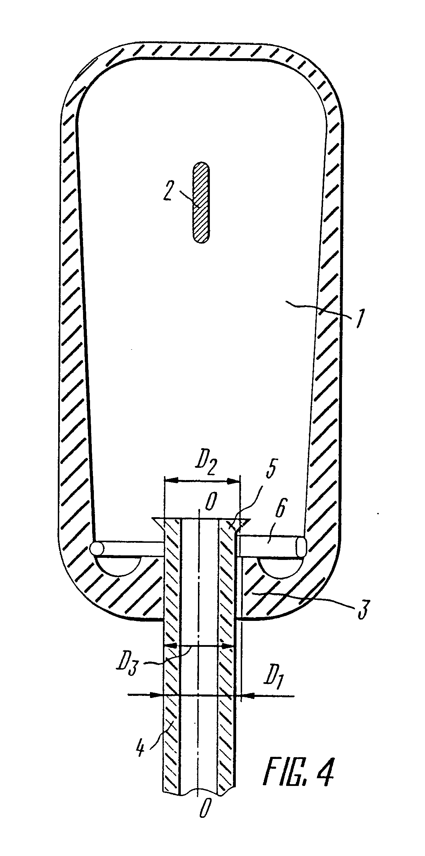

The bellmouth 5 of the catheter 4 of the occluding device may be provided with a sealing ring 6 (Fig. 2) encompassing the working end of the catheter 4 from the inner side of the balloon 1 (Fig. 2). Besides, the bellmouth 5 may be bevelled at an angle to a longitudinal axis 0-0 of the catheter 4. Fig. 3 presents the sealing ring 6, wherein Ref. No. 7 indicates an opening made in the sealing ring 6.

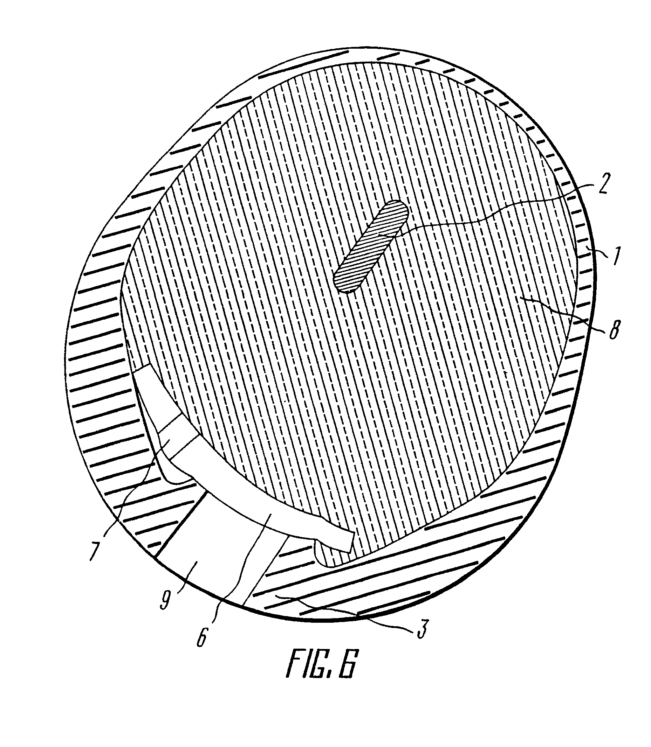

The balloon 1 (Fig. 6) introduced into the aneurysmal sac is filled with a rapidly solidifying matter 8. As a result, the balloon 1 is distended so as to fill the aneurysmal sac. Then, the catheter 4 (Fig. 4) is withdrawn from the sealing ring 6 through an opening 9 in the sphincter 3. The sealing ring 6 (Fig. 6) is forced by the rapidly solidifying matter 8 against the opening 9 in the sphincter 3. It is due to the bevelled end of the bellmouth 5 (Fig. 1) of the catheter 4 that the sealing ring 6 is displaced so that its self-sealing opening 7 gets out of register with the opening 9 in the balloon 1. The bevelled end of the bellmouth 5 of the catheter 4 practically rules out alignment of the openings 7 and 9 (Fig. 6) in the sealing ring 6 and the balloon 1, respectively. This provides for reliable closure of the balloon 1 containing the rapidly solidifying matter at the stage of incomplete polymerization thereof.

The occluding device as shown in Fig. 4 may feature rigid fixation of the sealing ring 6 on the inner surface of the sphincter 3. The opening 7 (Fig. 3) in the sealing ring 6 is offset with respect to the opening 9 in the sphincter 3 by an amount exceeding the diameter ($D_1$) of the latter, and the diameter $D_1$ of the opening in the sphincter 3 and the diameter $D_2$ of the opening 7 in the sealing ring 6 exceed the diameter $D_3$ of the catheter 4. The sealing ring 6 is rigidly fixable both at several points of its circumference and throughout the circumferential length thereof.

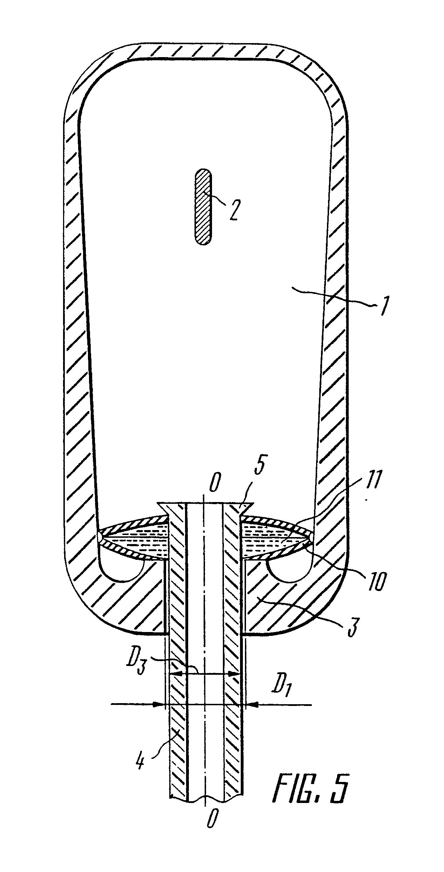

The occluding device of the invention may have a sealing disk 10 (Fig. 5), which encompasses the working end of the catheter 4 and is filled with a liquid 11. The balloon 1 inserted into the aneurysmal sac is filled with the rapidly solidifying matter 8, whereupon one should proceed with detaching the balloon 1 from the catheter 4. The catheter 4, while reciprocating, is readily wetted with the liquid 11 contained in the disk 10 and is easily removed from the balloon 1.

Having diagnosed, e.g., an arterial aneurysm, of the vessels of the internal carotid artery vascular channel, one should preselect a specific version of the occluding device, taking due account of its elasticity, an effort required to detach the catheter 4 from the balloon 1, and prevention of spontaneous detachment of the balloon 1 from the catheter 4 due to specific features of the blood flow along a given vascular channel. Let us assume that an occluding device having the sealing ring 6 (Fig. 4) rigidly fixed on the inner surface of the sphincter 3 has been selected for a given particular surgery.

Once the patient to be operated upon has been given preoperative management, the carotid artery is punctured with a special needle (omitted in the Drawing). Then, the occluding device is inserted into the carotid artery through the puncture needle and the balloon 1 is then brought to the pathological focus under roentgenotelevision monitoring. Once the balloon 1 has been introduced into the aneurysmal sac, the rapidly solidifying matter 8 (Fig. 6) is fed along the catheter 4 to fill the balloon 1 until the latter fills completely the aneurysmal sac. As a result, the sphincter 3 of the balloon 1 and the sealing ring 6 are expanded, the latter being fixed rigidly on the inner surface of the sphincter 3. The opening 9 in the sphincter 3 and the opening 7 in the sealing ring 6 get expended, too, thus reducing the force of adhesion of the balloon 1 with the catheter 4. An increasing gap between the inner face of the opening 9 in the sphincter 3 and the outer wall of catheter 4 enables the portion of the latter which is located in the opening, to be wetted with blood when an attempt is made to withdraw the catheter 4. Wetting of the bellmouth 5 of the catheter 4 reduces drastically the forces of friction of the catheter 4 against the faces of the openings 9 and 7, whereby the catheter 4 is easily withdrawn from the balloon 1. This done, the sealing ring 4 gets contrated so that its opening 7 is displaced with respect to the opening 9 in the sphincter 3, thus reliably closing the balloon 1 and preventing the rapidly solididying matter 8 against escaping therefrom and penetrating into the vascular channel.

Industrial Applicability

It is extremely valuable that in a majotity of cases the occluding device of the present invention is instrumental in a reconstructive vasoplastic surgery, that is, an ideal surgical procedure, namely, an occlusion of an arterial aneurysmal sac so as to exclude the aneurysm from the blood flow and retain the patency of the aneurysmatic cerebral artery and of the vessels of other organs. Reliable fixation of the balloon and catheter, low efforts required for their disengagement during occluding of aneurysms, all this makes it possible to considerably reduce the amount of postoperative complications caused by spontaneous removal of the balloon by the blood flow, tearing of the blood vessels off the cerebral tissues, as well as by rupture of vessels and aneurysms. A great deal of experience that has been amassed during many-year practice in treatment of patients suffering from arterial aneurysms demostrates an endovascular exclusion of such aneurysms to be most physiological, highly reliable and efficacious and involves but a minimized operation injury. It is preservation of the parent vessel and complete occlusion of the aneurysmal sac that are the fundamentally novel features of endovascular surgery for arterial aneurysms.

The occluding device as disclosed in the present invention is suitable for carrying out reconstructive vasoplastic surgery in various carotid-cavernous vasculostomies, successful exclusion of various arteriosinusal vasostomies, and treatment of vascular aneurysms of the abdominal organs, the neck and the aorta, etc.

Endovascular surgery with the aid of the detachable balloons-catheters of the invention performed for false traumatic aneurysms of the cavernous segment of the internal carotid artery, makes it possible to carry out a reconstructive surgical procedure. The present balloon-catheter is the sole surgical appliance suitable for performing such procedures in the case of the aforesaid pathology.

Use of ready-to-detach balloons and catheters enables one to successfully exclude diverse intracranial and extracranial arteriovenous aneurysms from the blood flow.

The occluding devices of the invention provides for reliable prevention of repeated hemorrhage due to possible recurrent ruptures of aneurysms and practically rules out ruptures of vascular aneurysmal walls, as well as tearing of vessels off organs and tissues.

**Claims**

1. An occluding device, comprising a detachable distensible balloon (1) provided with a radiopaque marker (2) and communicating, through a sphincter (3), with a catheter working end (4), **characterized** in that it is shaped as a bellmouth (5) whose flaring-out portion faces inwards the balloon (1).

2. An occluding device as claimed in Claim 1, **characterized** in that it comprises a sealing ring (6) encompassing the catheter working end (4) from the inner side of the balloon, the greater base of the bell-mouth (5) being bevelled at an angle to the catheter longitudinal axis.

3. An occluding device as claimed in Claim 2, **characterized** in that the sealing ring (6) is fixed in place on the inner surface of the sphincter (3), and the opening (7) in the ring is offset with respect to the opening in the sphincter (3) by an amount exceeding the sphincter diameter.

4. An occluding device as claimed in Claim 1, **characterized** in that it comprises a disk (10) encompassing the working end of the catheter (4) from the inner side of the balloon and filled with a liquid (11).

FIG. 1

FIG. 2          FIG. 3

FIG. 4

FIG. 5

FIG. 6

# INTERNATIONAL SEARCH REPORT

International Application No PCT/SU 89/00110

**I. CLASSIFICATION OF SUBJECT MATTER** (if several classification symbols apply, indicate all) *

According to International Patent Classification (IPC) or to both National Classification and IPC

IPC$^5$: A 61 M 25/00, A 61 B 17/12

**II. FIELDS SEARCHED**

Minimum Documentation Searched [7]

| Classification System | Classification Symbols |
|---|---|
| IPC$^4$ | A 61 M 25/00, A 61 B 17/12 |

Documentation Searched other than Minimum Documentation
to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | GB, B, 1333096 (JAMES ALEXANDER HUNTER) 10 October 1973 (10.10.73) figures 4,5,6,11 | 1-4 |
| A | SU, AI, 787037 (Nauchno-issledo vatelsky institut neirokhirurgii im N.N. Burdenko) 15 December 1980 (15.12.80), see the claims and the drawings | 1-4 |
| A | SU, AI, 787039, (institut neiro-khirurgii im N.N. Burdenko) 15 December 1980 (15.12.80) see the claims and the drawings | 1-4 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 05 December 1989 (05.12.89) | 18 January 1990 (18.01.90) |

| International Searching Authority | Signature of Authorized Officer |
|---|---|
| ISA/SU | |

Form PCT/ISA/210 (second sheet) (January 1985)